# EUROPEAN PATENT APPLICATION

(11) **EP 3 939 656 A1**
(43) Date of publication of application: **19.01.2022**
(21) Application number: 20770836.3
(22) Date of filing: 13.03.2020
(51) Int. Cl.: A61P 3/06, A61P 9/14, A61P 43/00, A61K 31/47, A61K 31/4704

(54) **PHARMACEUTICAL COMPOSITION FOR TREATING AORTIC ANEURYSM**

(30) Priority: 13.03.2019 JP 2019045333
(71) Applicant: National University Corporation Hamamatsu University School of Medicine, Hamamatsu-shi, Shizuoka 431-3192 (JP); KOWA COMPANY, LTD., Naka-ku Nagoya-shi Aichi 460-8625 (JP)
(72) Inventor: UNNO Naoki, Hamamatsu-shi, Shizuoka 431-3192 (JP); TANAKA Hiroki, Hamamatsu-shi, Shizuoka 431-3192 (JP); YATA Tatsuro, Hamamatsu-shi, Shizuoka 431-3192 (JP); KAYAMA Takafumi, Hamamatsu-shi, Shizuoka 431-3192 (JP); INOUE Keisuke, Higashimurayama-shi, Tokyo 189-0022 (JP); TOYA Kiyoshi, Tokyo 103-8433 (JP)
(74) Representative: Gerauer, Marc Philippé
(86) International application number: PCT/JP2020/011042
(87) International publication number: WO 2020/184703

(57) **Abstract**

The present invention addresses the problem of providing a therapeutic agent for an abdominal aortic aneurysm, the therapeutic agent making drug therapy possible.

The present invention provides a therapeutic agent for aortic aneurysm, the therapeutic agent having as active ingredients: (-)-6-[3-[3-cyclopropyl-3-[(1R,2R)-2-hydroxycyclohexyl]ureido]propoxy]-2(1H)-quinolinone or a salt thereof, or a solvate of these; and a statin.

## Description

### Technical Field

The present invention relates to a pharmaceutical composition for the treatment of an aortic aneurysm. In particular, the present invention relates to a pharmaceutical composition for the treatment of an abdominal aortic aneurysm.

### Background Art

An aortic aneurysm is defined as "a state in which part of an aortic wall dilates or protrudes (in diameter) whole circumferentially or locally". When part of the aortic wall dilates locally to form an aneurysm, or the diameter dilates to more than 1.5 times the normal diameter (45 mm for the thorax and 30 mm for the abdomen) (dilates in the form of a fusiform or sac), the state is called "aneurysm". The incidence of annual onset in Japan has been reported to be about 3 per 100,000 population, but according to the statistics of the Japanese Association for Thoracic Surgery, the number of aortic aneurysm surgery cases is increasing year by year.

Aortic aneurysms are classified depending on the form of their aneurysm wall, the location of the aneurysm, the cause, the shape of the aneurysm, or the like.

Aortic aneurysms are classified into true, false, and dissecting, depending on the form of their aneurysm wall.

Aortic aneurysms are classified into thoracic, thoracoabdominal, and abdominal, depending on the location of the aneurysm.

Aortic aneurysms are classified into atherosclerotic, traumatic, inflammatory, infectious, and congenital, depending on the cause.

In addition, depending on the shape of the aneurysm, aortic aneurysms are classified into fusiform type and saccular type.

An abdominal aortic aneurysm (hereinafter also referred to as "AAA") is a disease in which the aortic wall becomes fragile and dilates to 1.5 times or more the normal diameter (AAA maximum short-axis diameter: more than 30 mm), and the mortality rate when rupture occurs is said to be 50% to 90%. The rupture rate (%/year) of AAA is almost 0%/year when the AAA maximum short-axis diameter is about 30 mm, but it increases as the diameter increases, and when the diameter exceeds 70 mm, it is said to be 20% to 40%/year. There is no established drug treatment for AAAs at risk of rupture, and the only treatment option is surgery to prevent aneurysm rupture by aortic aneurysmectomy (blood vessel prosthesis implantation) or endovascular stent-grafting. A maximum AAA short-axis diameter of more than 50 mm or at least 5-mm dilatation within half a year is the measure for implementation of surgery (Reference: http://www.j-circ.or.jp/guideline/pdf/JCS2011_takamoto_d.pdf). However, AAAs are common in the elderly, and it is difficult to treat patients with poor general condition who cannot select aortic aneurysmectomy inseparable from laparotomy and patients who cannot adapt to stent-grafting because of morphological restrictions of aortic aneurysms. Therefore, there is a profound desire to develop a therapeutic agent for an aortic aneurysm by preventing or suppressing dilatation and rupture of the aneurysm.

Regarding the development of therapeutic agents for AAA, there are known studies using animal models such as Angiotensin II (AngII)-induced models created by continuous infusion of AngII in APO-E-deficient mice, elastase-induced models, and calcium chloride (CaCl₂)-induced models. However, no drugs have shown efficacy in clinical trials after verification of the efficacy in these animal models. Accordingly, there is no clinically available therapeutic agents up to the present day.

These facts may be suggesting the limitations of basic studies using the animal models. In other words, these animal models are those in which, among the intima, media, and adventitia that constitute the blood vessel wall, arteriosclerosis of the intima is caused or inflammation of the media is externally induced to form an aneurysm, and the aortic aneurysm thus induced merely constitutes part of the pathology of human AAA. In short, the animal models do not necessarily reflect pathological conditions of human aortic aneurysms in an authentic manner. Specifically, AAAs in the animal models show many differences from human AAAs such that aneurysms do not rupture (elastase-induced or CaCl₂-induced models), maximum short-axis diameters do not continuously dilate (AngII-induced, elastase-induced, or CaCl₂-induced models), and intraluminal thrombi do not adhere within the walls of aortic aneurysms (AngII-induced or CaCl₂-induced models) (Non-Patent Document 1).

Among the existing models, the AngII-induced model leads to rupture. However, in this model, an aneurysm is formed in the aortas in the thorax and just below the diaphragm rather than in the abdomen, so, considering that most of human AAA occurs in the aortas below the renal artery branch, the AngII-induced model does not directly mimic human AAA. Also, unlike human AAA in which part of an aortic wall dilates (or protrudes), this model presents an aortic dissection phenotype in which the aortic wall is separated into two layers and formed into two cavities along the artery running. Therefore, there are also differences in pathology. Therefore, the AngII-induced model cannot be said to be appropriate as an experimental model for evaluating AAA rupture. In addition, there are cases (Non-Patent Document 2) where the rupture could not be suppressed even when the aneurysm dilatation induced in the model animal was suppressed. Therefore, as far as the existing models are referred to, the suppression of AAA dilatation cannot always lead to suppress AAA rupture.

Under these circumstances, it has been found from the histopathological observation of human AAA that the AAA wall is ischemic and hypoxic due to the stenosis of vasa vasorum (VV), a microvessel that controls blood flow in the aortic wall. Evoking circulatory failure of the VV and hypoxia in the aortic walls enables development of a new animal model that produces a histopathological phenotype very similar to a human AAA histopathological phenotype as compared with models in the related art (Non-Patent Document 3). The aortic aneurysm histopathological phenotype of this model reproduces the intimal thickening and medial degeneration of the arterial wall, as well as the increase in intraluminal thrombi and increase in adventitial adipocytes, which are not observed in conventional animal models. This model has newly shown relevance between AAA rupture and adipogenesis in the vascular wall (Non-Patent Document 4). The present inventors have considered that the model (hereinafter may be referred to as "aortic wall hypoxia-induced rat AAA model") created from a rat enables evaluation of drug effect more appropriately than models in the related art.

(-)-6-[3-[3-cyclopropyl-3-[(1R,2R)-2-hydroxycyclohexyl]ureido]propoxy]-2(1H)-quinolinone (hereinafter may be referred to as "Compound 1") have antiplatelet action, antithrombotic action and PDE3 inhibitory action. Applicable scope of the Compound 1 includes brain diseases such as cerebral atherosclerosis, cerebral infarction, transient cerebral ischemic attack, and recovery ischemic neurological deficit; heart diseases such as myocardial infarction and angina pectoris; chronic arterial occlusion such as Buerger's disease, obstructive arteriosclerosis, and intermittent claudication; diabetic complications such as diabetic neuropathy, diabetic skin ulcer, and diabetic nephropathy; and ischemic diseases such as prevention of restenosis after percutaneous transluminal coronary angioplasty (PTCA), directional coronary atherectomy (DCA) and stent intervention, and the like, prevention of re-occlusion after transplantation of artificial organs such as artificial blood vessels, and kidneys, and the like, and prevention of thrombi and emboli development after surgery, and during extracorporeal circulation such as kidney dialysis (Non-Patent Document 5 and Patent Document 1). However, it is not known that Compound 1 is effective for the treatment of an aortic aneurysm, for example, treatment based on suppression of rupture or dilatation of an abdominal aortic aneurysm.

Meanwhile, statins known as HMG-CoA reductase inhibitors are widely used worldwide for the treatment of dyslipidemia. This class of drugs is particularly effective in lowering LDL-C levels and is believed to reduce the incidence of cardiovascular disease.

There have been several reports (Non-Patent Document 6 to 9) on the effects of statins on AAA, including the expected anti-inflammatory effects of statins, but another report (Non-Patent Document 10) points out that statins show inconsistent results in effect.

In addition, there is no known effect on AAAs when Compound 1 and a statin are used in combination.

### Citation List

### Patent Document

Patent Document 1: WO 97/12869

### Non-Patent Document

Non-Patent Document 1: Arterioscler. Thromb. Vasc. Biol. 2017 (Mar); 37(3): 401-10
Non-Patent Document 2: Atherosclerosis 2010; 210(1): 51-6
Non-Patent Document 3: PLoS ONE 2015; 10(8): e0134386
Non-Patent Document 4: Sci. Rep. 2016; 6:31268
Non-Patent Document 5: Biochem. Biophys. Research Commun. 2007; 353(4): 1111-14
Non-Patent Document 6: J. Vasc. Surg. 2006; 43(1): 117-24
Non-Patent Document 7: Ann. Surg. 2005; 241(1): 92-101
Non-Patent Document 8: Atherosclerosis 2009; 202(1): 34-40
Non-Patent Document 9: Int. J. Mol. Sci. 2015; 16(5): 11213-28
Non-Patent Document 10: Eur. J. Vasc. Endovasc. Surg. 2015; 50(6): 702-13

### Summary of the Invention

### Problems to be Solved by the Invention

An object of the present invention is to provide a therapeutic composition for an aortic aneurysm, particularly a therapeutic composition for suppressing rupture and dilatation of an abdominal aortic aneurysm.

### Means for Solving the Problems

As a result of intensive studies to solve the above-mentioned problems, the present inventors have found that a concomitant use of Compound 1 and a statin suppresses rupture of abdominal aortic aneurysms in aortic wall hypoxia-induced rat AAA models, and further suppresses dilatation thereof, and completed the present invention.

That is, the present invention relates to a pharmaceutical composition for treating an aortic aneurysm comprising a combination of (-)-6-[3-[3-cyclopropyl-3-[(1R,2R)-2-hydroxycyclohexyl]ureido]propoxy]-2(1H)-quinolinone (Compound 1) or a salt, or a solvate, or a solvate of the salt thereof, and a statin. More preferably, the present invention relates to a pharmaceutical composition for treating an abdominal aortic aneurysm comprising a combination of Compound 1 or a salt, or a solvate, or a solvate of the salt thereof, and a statin. In a further preferred aspect, the treatment of an abdominal aortic aneurysm of the present invention relates to suppression of aortic aneurysm rupture and/or suppression of dilatation of an aortic aneurysm, and the present invention relates to an agent for suppressing abdominal aortic aneurysm rupture and/or an agent for suppressing dilatation of an abdominal aortic aneurysm comprising a combination of Compound 1 or a salt, or a solvate, or a solvate of the salt thereof, and a statin. Here, the suppression of aortic aneurysm rupture can also be referred to as prevention of aortic aneurysm rupture. Therefore, the agent for suppressing (abdominal) aortic aneurysm rupture, which is one aspect of the present invention, can also be referred to as a prophylactic agent for (abdominal) aortic aneurysm rupture.

Moreover, the present invention relates to a pharmaceutical composition for the treatment for patients with aortic aneurysm after endovascular stent-grafting, as one preferable aspect. The pharmaceutical composition is used to treat an aortic aneurysm by rupture suppression, dilatation suppression, and/or size reduction. For example, the present invention relates to an agent for suppressing abdominal aortic aneurysm rupture after endovascular stent-grafting, comprising a combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin.

As another preferred aspect, the present invention relates to a pharmaceutical composition for the treatment for patients with aortic aneurysm before endovascular stent-grafting or aortic aneurysmectomy (blood vessel prosthesis implantation). At a stage before endovascular stent-grafting or aortic aneurysmectomy (blood vessel prosthesis implantation), the pharmaceutical composition of the present invention is used for the treatment of an aortic aneurysm (rupture suppression, dilatation suppression, and/or size reduction). For example, the present invention relates to an agent for suppressing abdominal aortic aneurysm rupture before endovascular stent-grafting or aortic aneurysmectomy (blood vessel prosthesis implantation), comprising a combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin.

That is, the present invention relates to a therapeutic application for an aortic aneurysm using a combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin. The present invention provides a composition comprising (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin. An aspect of the composition is a pharmaceutical composition. An aspect of the pharmaceutical composition is a pharmaceutical composition for treating an aortic aneurysm. More specifically, the present invention relates to the following [1] to [49].
[1] A pharmaceutical composition for the treatment of an aortic aneurysm comprising a combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin.
[2] The pharmaceutical composition according to above [1], wherein the treatment of the aortic aneurysm includes at least one treatment selected from the group consisting of suppression of aneurysm rupture, suppression of aneurysm dilatation, and aneurysm size reduction.
[3] The pharmaceutical composition according to above [1] or [2], wherein the treatment of the aortic aneurysm is suppression of aortic aneurysm rupture.
[4] The pharmaceutical composition according to above [1] or [2], wherein the treatment of the aortic aneurysm is suppression of dilatation of the aortic aneurysm.
[5] The pharmaceutical composition according to any one of above [1] to [4], which is used for patients with aortic aneurysm after endovascular stent-grafting.
[6] The pharmaceutical composition according to any one of above [1] to [4], which is used for patients with aortic aneurysm before endovascular stent-grafting or aortic aneurysmectomy (blood vessel prosthesis implantation).
[7] The pharmaceutical composition according to any one of above [1] to [6], wherein the aortic aneurysm is an abdominal aortic aneurysm.
[8] The pharmaceutical composition according to above [7], which is used for patients with abdominal aortic aneurysm having a maximum short-axis diameter of greater than 30 mm or greater than 40 mm.
[9] The pharmaceutical composition according to above [8], which is used for patients with abdominal aortic aneurysm having a maximum short-axis diameter of 50 mm or less.
[10] The pharmaceutical composition according to above [7] or [8], which is used for patients with abdominal aortic aneurysm having a maximum short-axis diameter of greater than 50 mm, greater than 60 mm, or greater than 70 mm.
[11] The pharmaceutical composition according to any one of above [1] to [10], which comprises a pharmaceutically acceptable carrier.
[12] The pharmaceutical composition according to any one of above [1] to [11], wherein the statin is one selected from the group consisting of pravastatin, simvastatin, fluvastatin, atorvastatin, pitavastatin, and rosuvastatin.
[13] The pharmaceutical composition according to any one of above [1] to [12], wherein the statin is pitavastatin or a salt, or a solvate, or a solvate of the salt thereof.
[14] The pharmaceutical composition for according to any one of above [1] to [13], wherein the statin is pitavastatin calcium or pitavastatin calcium hydrate.
[15] A method for treating an aortic aneurysm, comprising a step of administering of an effective amount of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin to a subject in need of treatment, wherein the subject is a patient with an aortic aneurysm selected from the group consisting of an abdominal aortic aneurysm, a thoracoabdominal aortic aneurysm, and a thoracic aortic aneurysm.
[16] The method according to above [15], wherein the treatment needed is a treatment selected from the group consisting of suppression of aortic aneurysm rupture, suppression of aortic aneurysm dilatation, and aortic aneurysm size reduction.
[17] The method according to above [15] or [16], wherein treatment needed is suppression of aortic aneurysm rupture.
[18] The method according to above [15] or [16], wherein the treatment needed is suppression of aortic aneurysm dilatation.
[19] The method according to any one of above [15] to [18], wherein the subject is a patient with an aortic aneurysm after endovascular stent-grafting.
[20] The method according to any one of above [15] to [18], wherein the subject is a patient with an aortic aneurysm before endovascular stent-grafting or aortic aneurysmectomy (blood vessel prosthesis implantation).
[21] The method according to any one of above [15] to [20], wherein the subject is a patient with an abdominal aortic aneurysm.
[22] The method according to above [21], wherein the subject is a patient with an abdominal aortic aneurysm having maximum short-axis diameter of greater than 30 mm or greater than 40 mm.
[23] The method according to above [22], wherein the subject is a patient with an abdominal aortic aneurysm having maximum short-axis diameter of 50 mm or less.
[24] The method according to above [21] or [22], wherein the subject is a patient with an abdominal aortic aneurysm having maximum short-axis diameter of greater than 50 mm, greater than 60 mm, or greater than 70 mm.
[25] The method according to any one of above [15] to [24], wherein the step is performed together with administration of a pharmaceutically acceptable carrier.
[26] The method according to any one of above [15] to [25], wherein the statin is one selected from the group consisting of pravastatin, simvastatin, fluvastatin, atorvastatin, pitavastatin, and rosuvastatin.
[27] The method according to any one of above [15] to [26], wherein the statin is pitavastatin or a salt, or a solvate, or a solvate of the salt thereof.
[28] The method according to any one of above [15] to [27], wherein the statin is pitavastatin calcium or pitavastatin calcium hydrate.
[29] Use of a combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin for the manufacture of a pharmaceutical composition for the treatment of an aortic aneurysm.
[30] The use according to above [29], wherein the treatment of the aortic aneurysm includes at least one treatment selected from the group consisting of suppression of aneurysm rupture, suppression of aneurysm dilatation, and aneurysm size reduction.
[31] The use according to above [29] or [30], wherein the treatment of the aortic aneurysm is suppression of aortic aneurysm rupture.
[32] The use according to above [29] or [30], wherein the treatment of the aortic aneurysm is suppression of aortic aneurysm dilatation.
[33] The use according to any one of above [29] to [32], which is used for patients with aortic aneurysm after endovascular stent-grafting.
[34] The use according to any one of above [29] to [32], which is used for patients with aortic aneurysm before endovascular stent-grafting or aortic aneurysmectomy (blood vessel prosthesis implantation).
[35] The use according to any one of above [29] to [34], wherein the aortic aneurysm is an abdominal aortic aneurysm.
[36] The use according to any one of above [29] to [35], wherein the pharmaceutical composition comprises a pharmaceutically acceptable carrier.
[37] The use according to any one of above [29] to [36], wherein the statin is one selected from the group consisting of pravastatin, simvastatin, fluvastatin, atorvastatin, pitavastatin, and rosuvastatin.
[38] The use according to any one of above [29] to [37], wherein the statin is pitavastatin or a salt, or a solvate, or a solvate of the salt thereof.
[39] The use according to any one of above [29] to [38], wherein the statin is pitavastatin calcium or pitavastatin calcium hydrate.
[40] A combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin for use in the treatment of an aortic aneurysm.
[41] The combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin for use according to above [40], wherein the treatment of the aortic aneurysm includes at least one treatment selected from the group consisting of suppression of aneurysm rupture, suppression of aneurysm dilatation, and aneurysm size reduction.
[42] The combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin for use according to above [40] or [41], wherein the treatment of the aortic aneurysm is suppression of aortic aneurysm rupture.
[43] The combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin for use according to above [40] or [41], wherein the treatment of the aortic aneurysm is suppression of aortic aneurysm dilatation.
[44] The combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin for use according to any one of above [40] to [43], wherein the treatment is treatment for patients with aortic aneurysm after endovascular stent-grafting.
[45] The combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin for use according to any one of above [40] to [43], wherein the treatment is treatment for patients with aortic aneurysm before endovascular stent-grafting or aortic aneurysmectomy (blood vessel prosthesis implantation).
[46] The combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin for use according to above any one of [40] to [45], wherein the aortic aneurysm is an abdominal aortic aneurysm.
[47] The combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin for use according to any one of above [40] to [46], wherein the statin is one selected from the group consisting of pravastatin, simvastatin, fluvastatin, atorvastatin, pitavastatin, and rosuvastatin.
[48] The combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin for use according to any one of above [40] to [47], wherein the statin is pitavastatin or a salt, or a solvate, or a solvate of the salt thereof.
[49] The combination of (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin for use according to any one of above [40] to [48], wherein the statin is pitavastatin calcium or pitavastatin calcium hydrate.

The present invention also relates to the following [A] to [G].
[A] An agent for the treatment of an aortic aneurysm comprising as active ingredients: (-)-6-[3-[3-cyclopropyl-3-[(1R,2R)-2-hydroxycyclohexyl]ureido]propoxy]-2(1H)-quinolinone or a salt, or a solvate, or a solvate of the salt thereof; and a statin.
[B] The agent according to above [A], wherein the treatment of the aortic aneurysm includes at least one treatment selected from the group consisting of suppression of aneurysm rupture, suppression of aneurysm dilatation, and aneurysm size reduction.
[C] An agent for suppressing aortic aneurysm rupture comprising as active ingredients: (-)-6-[3-[3-cyclopropyl-3-[(1R,2R)-2-hydroxycyclohexyl]ureido]propoxy]-2(1H)-quinolinone or a salt, or a solvate, or a solvate of the salt thereof; and a statin.
[D] The agent according to above [C], wherein the aortic aneurysm is an abdominal aortic aneurysm.
[E] The agent according to above [C] or [D] wherein the aortic aneurysm rupture is aneurysm rupture after endovascular stent-grafting.
[F] An agent for suppressing aortic aneurysm dilatation comprising as active ingredients: (-)-6-[3-[3-cyclopropyl-3-[(1R,2R)-2-hydroxycyclohexyl]ureido]propoxy]-2(1H)-quinolinone or a salt, or a solvate, or a solvate of the salt thereof; and a statin.
[G] The agent according to above [F], wherein the aortic aneurysm is an abdominal aortic aneurysm.

### Effects of the Invention

A concomitant use of Compound 1 and a statin according to the present, as will be illustrated in the Examples below, suppressed AAA rupture and improved the non-rupture survival rate in an aortic wall hypoxia-induced rat AAA model. Furthermore, the dilatation of the AAA diameter was suppressed in the aortic wall hypoxia-induced rat AAA model. Therefore, (-)-6-[3-[3-cyclopropyl-3-[(1R,2R)-2-hydroxycyclohexyl]ureido]propoxy]-2(1H)-quinolinone or a salt, or a solvate, or a solvate of the salt thereof, and a statin according to the present invention can provide a novel medical treatment method for an aortic aneurysm typified by suppression of abdominal aortic aneurysm rupture and/or suppression of dilatation of the abdominal aortic aneurysm.

### Brief Description of Drawings

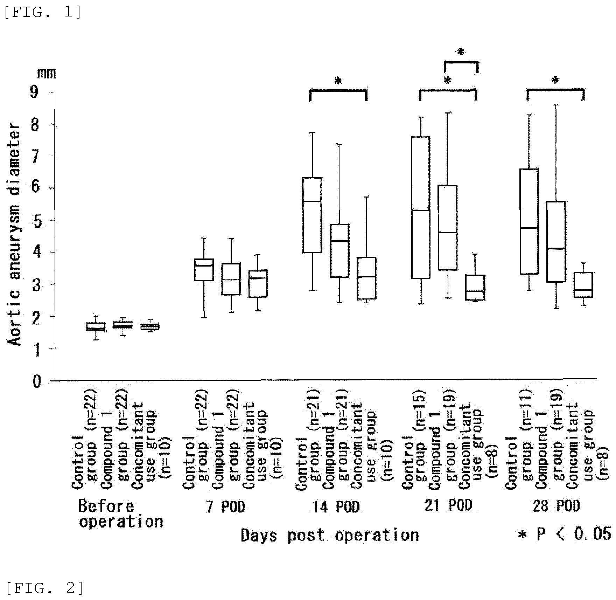

FIG. 1 shows the change of the aneurysm diameters (AAA maximum short-axis diameters) over days when aortic wall hypoxia-induced rat AAA model was fed with a normal diet, a Compound 1-mixed diet, or a mixed diet containing Compound 1 and pitavastatin calcium from one week before the AAA model creation operation(7 days before operation). The vertical axis indicates the aneurysm diameter, and the horizontal axis indicates the number of days elapsed after operation of AAA induction treatment (POD means postoperative days). Box plots show the minimum, the first quartile, the median, the third quartile, and the maximum (mm) of aneurysm diameters of each group. The number of samples (n) differs depending on time points since survival cases are measured at each time point. All rats survived by the measurement of the aneurysm diameter on Day 7 post operation, but the aneurysm diameter of the dead rats was not measured after Day 14 post operation. In the figure, the asterisk (*) indicates that when p-value was less than 0.05 by the Kruskal-Wallis test for each time point, and a significant difference of p < 0.05 was also observed by the Dunn-Bonferroni test between the groups.

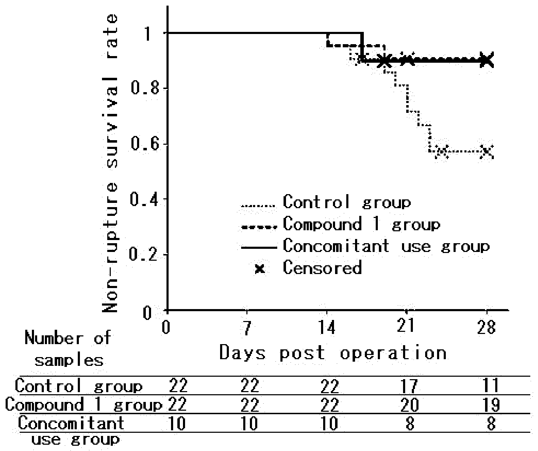

Fig. 2 shows the non-rupture survival rate when an aortic wall hypoxia-induced rat AAA model was fed with a normal diet, Compound 1-mixed diet or a mixed diet containing Compound 1 and pitavastatin calcium from 1 week before the AAA model creation operation (7 days before operation). The vertical axis indicates the cumulative survival (the survival at the time of induction treatment of the aortic wall hypoxia-induced rat AAA model is 1.0), and the horizontal axis indicates the number of days elapsed after operation (the number of days elapsed after treatment with the induction treatment day being Day 0). FIG. 2 shows survival functions of a control group (n = 22), a Compound 1 group (n = 22), and a concomitant use group (n = 10). The death of unknown cause identified during the observation and the sacrificial death due to the completion of the 28-day observation are censored (×) since those cases do not correspond to either the death caused by rupture or the survival, and those cases are excluded from the subsequent calculation of survival rates and rupture rates. In the table in the lower part of FIG. 2, the number of samples represents survival cases at the start of each time point. Since some samples died before the measurement of aneurysm diameters at each time point, the number of samples is different from that in FIG. 1.

### Modes for Carrying Out the Invention

Compound 1 of the present invention is a known compound and can be manufactured by the method disclosed in Patent Document 1 (WO 97/12869) or a method conforming thereto.

In the present invention, a salt or a solvate of Compound 1 can also be employed. The salt and solvate of Compound 1 can be manufactured by a conventional procedure.

The salt of Compound 1 of the present invention includes an addition salt with a base, and is not particularly limited as long as it is pharmaceutically acceptable. Examples of such salts include base addition salts with alkali metals such as potassium and sodium, and alkaline earth metals such as magnesium and calcium.

Examples of the solvate of Compound 1 of the present invention include hydrates and alcohol solvates (for example, ethanol solvates).

A dose of Compound 1 or a salt, or a solvate, or a solvate of the salt thereof is not particularly limited and varies depending on, for example, patient's body weight, age, sex, symptom, etc., and can be appropriately set by those skilled in the art. For an adult, Compound 1 is preferably administered in an amount of usually from 0.01 to 1000 mg, preferably from 5 to 400 mg, more preferably from 25 to 200 mg per day in 1 or 2 divided dose(s).

Herein, "statin" is an HMG-CoA reductase inhibitor. Examples of the statin include pravastatin, simvastatin, fluvastatin, atorvastatin, pitavastatin, rosuvastatin, and a salt, a solvate, and a solvate of the salt of each statin. Preferred examples of the statin include pitavastatin, a salt, a solvate, and a solvate of the salt thereof. In addition, pravastatin, simvastatin, fluvastatin, atorvastatin, pitavastatin, rosuvastatin, and a salt, a solvate and a solvate of the salt of each statin are commercially available.

Herein, "pravastatin, a salt, a solvate, or a solvate of the salt thereof" includes not only pravastatin itself but also a pharmaceutically acceptable salt of pravastatin (for example, alkali metal salts such as sodium salts and potassium salts; alkaline earth metal salts such as calcium salts and magnesium salts; organic amine salt such as phenethylamine salts; and ammonium salts), a solvate of pravastatin and a solvate of a pharmaceutically acceptable salt of pravastatin, wherein the solvate includes hydrates and alcohol solvates. One kind of these examples may be used singly, or two or more kinds may be used in combination.

A preferred example of pravastatin, a salt, a solvate, or a solvate of the salt thereof is pravastatin sodium (chemical name: Monosodium(3R,5R)-3,5-dihydroxy-7-{(1S,2S,6S,8S,8aR)-6-hydroxy-2-methyl-8[-(2S)-2-methylbutanoyloxy]-1,2,6,7,8,8a-hexahydronaphthalen-1-yl}heptanoate).

Pravastatin, a salt, a solvate, or a solvate of the salt thereof is a known compound and can be manufactured, for example, by the methods disclosed in JP S57-2240 A and US 4346227.

A dose of pravastatin or a salt, or a solvate, or a solvate of the salt thereof is not particularly limited and varies depending on, for example, recipient's sex, age, symptom, etc., and can be appropriately determined. For example, recipients preferably take pravastatin sodium in a dose of 1 to 160 mg, more preferably 2 to 120 mg, and particularly preferably 5 to 80 mg per day in 1 or 2 or more divided dose(s).

Herein, "simvastatin, a salt, a solvate, or a solvate of the salt thereof" includes not only simvastatin itself but also a pharmaceutically acceptable salt of simvastatin (for example, alkali metal salts such as sodium salts and potassium salts; alkaline earth metal salts such as calcium salts and magnesium salts; organic amine salt such as phenethylamine salts; and ammonium salts), a solvate of simvastatin and a solvate of a pharmaceutically acceptable salt of simvastatin, wherein the solvate includes hydrates and alcohol solvates One kind of these examples may be used singly, or two or more kinds may be used in combination.

A preferred example of simvastatin, a salt, a solvate, or a solvate of the salt thereof is simvastatin (chemical name: (1S,3R,7S,8S,8aR)-8-[2-[(2R,4R)-4-hydroxy-6-oxotetrahydro-2H-pyran-2-yl]ethyl]-3,7-dimethyl-1,2,3,7,8,8a-hexahydronaphthalen-1-yl 2,2-dimethylbutanoate).

Simvastatin, a salt, a solvate, or a solvate of the salt thereof is a known compound and can be manufactured, for example, by the method disclosed in US 4444784.

A dose of simvastatin or a salt, or a solvate, or a solvate of the salt thereof is not particularly limited and varies depending on, for example, recipient's sex, age, symptom, etc., and can be appropriately determined. For example, recipients preferably take simvastatin (free base)in a dose of 1 to 160 mg, more preferably 2 to 120 mg, and particularly preferably 5 to 80 mg per day in 1 or 2 or more divided dose(s).

Herein, "fluvastatin, a salt, a solvate, or a solvate of the salt thereof" includes not only fluvastatin itself but also a pharmaceutically acceptable salt of fluvastatin (for example, alkali metal salts such as sodium salts and potassium salts; alkaline earth metal salts such as calcium salts and magnesium salts; organic amine salt such as phenethylamine salts; and ammonium salts), a solvate of fluvastatin and a solvate of a pharmaceutically acceptable salt of fluvastatin, wherein the solvate includes hydrates and alcohol solvates One kind of these examples may be used singly, or two or more kinds may be used in combination.

A preferred example of fluvastatin, a salt, a solvate, or a solvate of the salt thereof is fluvastatin sodium (chemical name: (±)-(3RS,5SR,6E)-sodium-7-[3-(4-fluorophenyl)-1-(1-methylethyl)-1H-indol-2-yl]-3,5-dihydroxy-6-heptenoate).

Fluvastatin, a salt, a solvate, or a solvate of the salt thereof is a known compound and can be manufactured, for example, by the methods disclosed in JP S60-500015 A and US 5354772.

A dose of fluvastatin or a salt, or a solvate, or a solvate of the salt thereof is not particularly limited and varies depending on, for example, recipient's sex, age, symptom, etc., and can be appropriately determined.. For example, recipients preferably take fluvastatin (free base) in a dose of 1 to 160 mg, more preferably 5 to 120 mg, and particularly preferably 10 to 80 mg per day in 1 or 2 or more divided dose(s).

Herein, "atorvastatin, a salt, a solvate, or a solvate of the salt thereof" includes not only atorvastatin itself but also a pharmaceutically acceptable salt of atorvastatin (for example, alkali metal salts such as sodium salts and potassium salts; alkaline earth metal salts such as calcium salts and magnesium salts; organic amine salt such as phenethylamine salts; and ammonium salts), a solvate of atorvastatin and a solvate of a pharmaceutically acceptable salt of atorvastatin, wherein the solvate includes hydrates and alcohol solvates One kind of these examples may be used singly, or two or more kinds may be used in combination.

A preferred example of atorvastatin, a salt, a solvate, or a solvate of the salt thereof is atorvastatin calcium hydrate (chemical name: (-)-Monocalcium bis{(3R,5R)-7-[2-(4-fluorophenyl-5-isopropyl-3-phenyl-4-phenylcarbamoyl-1H-pyrrol-1-yl)-3,5-dihydroxyheptanoate]trihydrate}).

Atorvastatin, a salt, a solvate, or a solvate of the salt thereof is a known compound and can be manufactured, for example, by the methods disclosed in JP H3-58967 A and US 5273995.

A dose of atorvastatin or a salt, or a solvate, or a solvate of the salt thereof is not particularly limited and varies depending on, for example, recipient's sex, age, symptom, etc., and can be appropriately determined. For example, recipients preferably take atorvastatin (free base) in a dose of 1 to 160 mg, more preferably 2 to 120 mg, and particularly preferably 5 to 80 mg per day in 1 or 2 or more divided dose(s).

Herein, "pitavastatin, a salt, a solvate, or a solvate of the salt thereof" includes not only pitavastatin itself but also a pharmaceutically acceptable salt of pitavastatin (for example, alkali metal salts such as sodium salts and potassium salts; alkaline earth metal salts such as calcium salts and magnesium salts; organic amine salt such as phenethylamine salts; and ammonium salts), a solvate of pitavastatin and a solvate of a pharmaceutically acceptable salt of pitavastatin, wherein the solvate includes hydrates and alcohol solvates. One kind of these examples may be used singly, or two or more kinds may be used in combination.

A preferred example of pitavastatin, a salt, a solvate, or a solvate of the salt thereof is a calcium salt of pitavastatin or a hydrate thereof, and a more preferred example of pitavastatin is pitavastatin calcium (chemical name: (+)-monocalcium bis{(3R,5S,6E)-7-[2-cyclopropyl-4-(4-fluorophenyl)-3-quinolyl]-3,5-dihydroxy-6-heptenoate}) or a hydrate thereof (particularly pentahydrate thereof).

Pitavastatin, a salt, a solvate, or a solvate of the salt thereof is a known compound and can be manufactured, for example, by the methods disclosed in JP H1-279866 A and US 5856336.

A dose of pitavastatin or a salt, or a solvate, or a solvate of the salt thereof is not particularly limited and varies depending on, for example, recipient's sex, age, symptom, etc., and can be appropriately determined. For example, recipients preferably take pitavastatin calcium in a dose of 0.1 to 16 mg, more preferably 0.5 to 8 mg, and particularly preferably 1 to 4 mg per day in 1 or 2 or more divided dose(s).

Herein, "rosuvastatin, a salt, a solvate, or a solvate of the salt thereof" includes not only rosuvastatin itself but also a pharmaceutically acceptable salt of rosuvastatin (for example, alkali metal salts such as sodium salts and potassium salts; alkaline earth metal salts such as calcium salts and magnesium salts; organic amine salt such as phenethylamine salts; and ammonium salts), a solvate of rosuvastatin and a solvate of a pharmaceutically acceptable salt of rosuvastatin, wherein the solvate includes hydrates and alcohol solvates. One kind of these examples may be used singly, or two or more kinds may be used in combination.

A preferred example of rosuvastatin, a salt, a solvate, or a solvate of the salt thereof is rosuvastatin calcium (chemical name: Monocalcium bis ((3R,5S,6E)-7-{4-(4-fluorophenyl)-6-isopropyl-2-[methanesulfonyl(methyl)amino]pyrimidin-5-yl}-3,5-dihydroxyhept-6-enoate)).

Rosuvastatin, a salt, a solvate, or a solvate of the salt thereof is a known compound and can be manufactured, for example, by the methods disclosed in JP H5-178841 A and US 5260440.

A dose of rosuvastatin or a salt, or a solvate, or a solvate of the salt thereof is not particularly limited and varies depending on, for example, recipient's sex, age, symptom, etc., and can be appropriately determined. For example, recipients preferably take rosuvastatin (free base) in a dose of 0.5 to 80 mg, more preferably 1 to 60 mg, and particularly preferably 2.5 to 40 mg per day in 1 or 2 or more divided dose(s).

As the statin, pitavastatin, a salt, a solvate, or a solvate of the salt thereof is particularly preferable from a viewpoint of obtaining an excellent therapeutic action for an aortic aneurysm when the statin is combined with Compound 1, a salt, a solvate, or a solvate of the salt thereof. As is clear from Examples described later, a concomitant use of pitavastatin, a salt, a solvate, or a solvate of the salt thereof as the statin and Compound 1, a salt, a solvate, or a solvate of the salt thereof exhibits an excellent suppressive action on an increase in aneurysm diameter.

The agent for suppressing an abdominal aortic aneurysm rupture of the present invention suppresses AAA rupture, and thus prevents death from AAA rupture. Further, the agent can be applied for suppressing rupture of an aortic aneurysm including a thoracoabdominal aortic aneurysm and a thoracic aortic aneurysm. It is useful as an agent for suppressing aortic aneurysm rupture before aortic aneurysmectomy (blood vessel prosthesis implantation), before or after endovascular stent-grafting. The pharmaceutical composition for the treatment of an aortic aneurysm of the present invention is preferably used as an agent for suppressing AAA rupture before or after endovascular stent-grafting. Furthermore, the dilatation suppressing action of the pharmaceutical composition for the treatment of an abdominal aortic aneurysm of the present invention suppresses AAA dilatation, and thus prevents death from rupture resulting from the AAA dilatation. In addition, the pharmaceutical composition of the present invention can also be applied for suppressing dilatation of an aortic aneurysm including a thoracoabdominal aortic aneurysm and a thoracic aortic aneurysm. It is useful as an agent for suppressing aortic aneurysm dilatation before aortic aneurysmectomy (blood vessel prosthesis implantation), before or after endovascular stent-grafting. The pharmaceutical composition for the treatment of an aortic aneurysm of the present invention is preferably also used as an agent for suppressing AAA dilatation before or after endovascular stent-grafting.

A combination ratio of Compound 1, a salt, a solvate, or a solvate of the salt thereof, and a statin is not particularly limited and varies depending on, for example, recipient's sex, age, symptom, etc., and can be appropriately determined.. In order to obtain an excellent aortic aneurysm therapeutic action, it is preferable to combine 0.0001 to 16000 parts by mass, more preferably 0.00125 to 24 parts by mass, and particularly preferably 0.005 to 3.2 parts by mass of a statin (free base) with respect to 1 part by mass of Compound 1 (free base). Particularly, when using pitavastatin, a salt, a solvate, or a solvate of the salt thereof as the statin, it is preferable to combine 0.0001 to 1600 parts by mass, more preferably 0.00125 to 1.6 parts by mass, and particularly preferably 0.005 to 0.16 parts by mass of pitavastatin, a salt, a solvate, or a solvate of the salt thereof (free base) with respect to 1 part by mass of Compound 1 (free base) from the viewpoint of obtaining an excellent aortic aneurysm therapeutic action.

An agent for the treatment of an aortic aneurysm comprising a combination of Compound 1, a salt, a solvate, or a solvate of the salt thereof and a statin may separately include Compound 1, the salt, the solvate, or the solvate of the salt thereof as a component of the agent, and the statin as an another component of the agent as independent preparations (for example, the agent may be a package (kit) including a combination of a preparation comprising Compound 1, the salt, the solvate, or the solvate of the salt thereof and a preparation comprising the statin). Those preparations may be administered simultaneously or at intervals or may be administered as a pharmaceutical preparation (combination drug) containing both of the components. For sake of simplicity, it is preferable to administer a combination drug containing both of the components.

An agent for the treatment of an aortic aneurysm comprising a statin to be administered in combination with Compound 1, a salt, a solvate, or a solvate of the salt thereof is used together with Compound 1, the salt, the solvate, or the solvate of the salt thereof. For example, a medicament comprising the statin may be administered simultaneously with a medicament comprising Compound 1, the salt, the solvate, or the solvate of the salt thereof or at intervals.

A specific aspect of the medicament is, for example, one used for the treatment of an aortic aneurysm, comprising the following (A) and (B):
(A) a medicament comprising a statin for use in the treatment of an aortic aneurysm; and
(B) an instruction for administering the medicament in combination with Compound 1 or a salt, or a solvate, or a solvate of the salt thereof. Specific examples of the instruction include labels and what is called package inserts (package leaflet) with comments on effect/efficacy and dosage/administration.

In addition, an agent for the treatment of an aortic aneurysm comprising Compound 1 or a salt, or a solvate, or a solvate of the salt thereof to be administered in combination with a statin is used together with the statin. For example, a medicament comprising Compound 1 or a salt, or a solvate, or a solvate of the salt thereof is preferably administered simultaneously with a medicament comprising the statin or at intervals.

A specific aspect of the medicament is, for example, one used for the treatment of an aortic aneurysm, comprising the following (A) and (B):
(A) a medicament containing a Compound 1 or a salt, or a solvate, or a solvate of the salt thereof used for the treatment of an aortic aneurysm; and
(B) an instruction for administering the medicament in combination with a statin. Specific examples of the instruction include labels and what is called package inserts (package leaflet) with comments on effect/efficacy and dosage/administration.

Herein, the medicament is not particularly limited to have a specific shape (dosage form) and may be formed into any of solid preparation, semi-solid preparation, or liquid preparation. The dosage form can be selected according to the intended use or the like. Examples of the dosage form of the medication include those described in, for example, The Japanese Pharmacopoeia 17th edition, General Rules for Preparations. More specifically, examples of the dosage form for oral administration include solid preparations such as tablets (including, for example, normal tablets, orally disintegrating tablets, chewable tablets, effervescent tablets, dispersible tablets, and soluble tablets), capsules, granules (including, for example, effervescent granules), and powders; semi-solid preparations such as jellies for oral administration; liquid preparations such as oral liquid preparations (including, for example, elixirs, suspensions, emulsions, and lemonades). Examples of the dosage form for parenteral administration include injections, inhalations, preparations for ophthalmic application, preparations for ear drop application, preparations for nasal application, enemas, liniments, lotions, sprays, ointments, creams, gels, and patches.

For sake of simplicity, preferred examples of the dosage form of the medicament are solid preparations for oral administration. Particularly, tablets, capsules, granules, or powders are preferred examples.

The medicament can be produced depending on dosage forms by the known methods described in, for example, The Japanese Pharmacopoeia 17th edition, General Rules for Preparations. In this case, a pharmaceutically acceptable carrier (additive) may be added to the medicament. Examples of such additives include, but are not limited to, vehicles, binders, extenders, disintegrators, surfactants, lubricants, dispersants, buffers, preservatives, flavoring agents, fragrances, coatings, and diluents.

### Examples

Hereinafter, the present invention will be described more specifically with reference to Examples, but the present invention is in no way limited by the following Examples.

### Example 1 Effect on Aortic Wall Hypoxia-induced Rat AAA Model

The action of Compound 1 and pitavastatin in rat AAA models (Non-Patent Document 3) induced by operation were examined. In the operation, the aortic wall of each model was separated from the surrounding tissues. After a catheter was inserted, the abdominal aorta was ligated together with a catheter with a thread was studied.

In the test, Compound 1 was manufactured according to the method disclosed in Patent Document 1 and mixed, in a proportion of 0.15%, with a normal diet (FR2, manufactured by Funabashi Farm Co., Ltd.), and the Compound 1-mixed diet was used. In regard to pitavastatin, 0.003% of pitavastatin calcium was mixed with a normal diet(FR2, manufactured by Funabashi Farm Co., Ltd.) and the pitavastatin-mixed diet was used.

### (1) Test Animal and Rearing Environment

Sprague-Dawley male rats (300 to 350 g: Japan SLC, Inc.) were used in the experiment. The rats were reared at room temperature of 25°C ± 1°C, and allowed to freely take the feed and water.

### (2) Creation of Aortic Wall Hypoxia-induced Rat AAA Model

The model was created according to the method disclosed in Non-Patent Document 3. Specifically, a rat AAA model was created by separating the rat aortic wall from the surrounding tissue, inserting a polyurethane catheter, and then ligating the abdominal aorta together with a plastic catheter with a monofilament thread, and inducing AAA.

### (3) Grouping and Drug Administration

The rats were divided into the following groups: a group receiving a normal diet(hereinafter referred to as "control group") (n = 22); a group receiving a 0.15% Compound 1-mixed diet (hereinafter referred to as "Compound 1 group") (n = 22); and a group receiving 0.15% Compound 1-mixed diet and 0.003% pitavastatin calcium-mixed diet (hereinafter referred to as "concomitant use group") (n = 10). The rats were freely fed with the normal diet or either mixed feed in a period from one week before the AAA model creation operation (7 days before operation) to the end of the experiment.

### (4) Observation and Inspection Method

In each group, up to 28 days post operation, the aneurysm diameter was measured by echo and comparison in survival was made.

In addition, the surviving animals were sacrificed after 28 days to analyze the AAA pathological tissue.

The AAA maximum short-axis diameter was measured with an ultrasonic diagnostic apparatus (Vevo770, manufactured by VisualSonics Inc.) on the day of operation and Day 7, Day 14, Day 21 and Day 28 post operation.

### (5) Result

FIG. 1 shows measurement results of the aneurysm diameters.

In the concomitant use group, dilatation of aneurysm diameter was suppressed as compared with the control group and the Compound 1 group. Particularly, in the concomitant use group, an increase in aneurysm diameter was significantly suppressed as compared with the control group on Day 14 post operation, the control group and the Compound 1 group on Day 21 post operation, and the control group on Day 28 post operation (on each day, p < 0.05).

Next, the results of the non-rupture survival rates are shown in Fig. 2.

In the follow-up in a period from the day of operation to Day 28 post operation, the mortality rates due to rupture in the Compound 1 group and the concomitant use group were found lower than that in the control group. In other words, the former groups showed a trend toward improvement in survival rate. Note that one unexplained death case in the Compound 1 group, two unexplained death cases in the control group, and one unexplained death case in the concomitant use group were found during the period of observation and that these cases were censored and excluded from the subsequent calculation of survival rates and rupture rates. All the other death cases were confirmed, by autopsy, to be caused by AAA rupture.

These results clearly show that a concomitant use of Compound 1 and a statin is extremely effective in suppressing the rupture and dilatation of AAAs and is extremely useful as a pharmaceutical composition for the treatment of an aortic aneurysm, particularly, as a rupture inhibitor for AAAs (preventive agent for AAA rupture) and an agent for suppressing AAA dilatation (preventive agent for AAA dilatation).

### Industrial Applicability

A concomitant use of Compound 1 and a statin according to the present invention is effective for suppressing rupture and/or dilatation of an aortic aneurysm and has industrial applicability as a medical treatment for an aortic aneurysm.

## Claims

1. An agent for the treatment of an aortic aneurysm comprising, as active ingredients: (-)-6-[3-[3-cyclopropyl-3-[(1R,2R)-2-hydroxycyclohexyl]ureido]propoxy]-2(1H)-quinolinone or a salt, or a solvate, or a solvate of the salt thereof; and a statin.

2. The agent according to claim 1, wherein the treatment of the aortic aneurysm includes at least one treatment selected from the group consisting of suppression of aneurysm rupture, suppression of aneurysm dilatation, and aneurysm size reduction.

3. An agent for suppressing aortic aneurysm rupture comprising, as active ingredients: (-)-6-[3-[3-cyclopropyl-3-[(1R,2R)-2-hydroxycyclohexyl]ureido]propoxy]-2(1H)-quinolinone or a salt, or a solvate, or a solvate of the salt thereof; and a statin.

4. The agent according to claim 3, wherein the aortic aneurysm is an abdominal aortic aneurysm.

5. The agent according to claim 3 or 4, wherein the aortic aneurysm rupture is aortic aneurysm rupture after endovascular stent-grafting.

6. An agent for suppressing aortic aneurysm dilatation comprising, as active ingredients: (-)-6-[3-[3-cyclopropyl-3-[(1R,2R)-2-hydroxycyclohexyl]ureido]propoxy]-2(1H)-quinolinone or a salt, or a solvate, or a solvate of the salt thereof; and a statin.

7. The agent according to claim 6, wherein the aortic aneurysm is an abdominal aortic aneurysm.
